# EUROPEAN PATENT SPECIFICATION

(11) **EP 0 830 204 B1**
(45) Date of publication and mention of the grant of the patent: **10.02.1999**
(21) Application number: 96909708.8
(22) Date of filing: 14.03.1996
(51) Int. Cl.: B01J 35/02, C07C 17/156, C07C 19/045

(54) **METHOD OF OXYCHLORINATION**
VERFAHREN ZUR OXYCHLORIERUNG
PROCEDE D'OXYCHLORATION

(30) Priority: 07.06.1995 US 477492
(43) Date of publication of application: 25.03.1998
(73) Proprietor: THE DOW CHEMICAL COMPANY, Midland, Michigan 48674 (US)
(72) Inventor: REED, Daniel, J., Angleton, TX 77515 (US); GONZALEZ, Jorge, A., Houston, TX 77059 (US); SALINAS, Leopoldo, III, Lake Jackson, TX 77566 (US); MORRIS, Thomas, E., Lake Jackson, TX 77566 (US)
(74) Representative: Patentanwälte Sternagel & Fleischer
(86) International application number: PCT/US96/03541
(87) International publication number: WO 96/40431

(56) References cited:
- EP-A- 0 141 997
- EP-A- 0 146 925
- EP-A- 0 461 331
- EP-A- 0 678 331
- DE-A- 1 468 465
- GB-A- 1 218 413

## Description

The present invention relates to a catalyst and to a method of oxychlorination. In another aspect, the present invention relates to hollow cylindrically shaped catalyst having vanes within the hollow cylinder, and to a method of oxychlorination utilizing a split feed of oxygen and hydrochloric acid to a two reactor system.

1,2-dichloroethane, commonly known as ethylene dichloride ("EDC") is a compound manufactured industrially on a scale of several million tons per year, which, on pyrolysis is converted into vinyl chloride monomer and hydrochloric acid. Vinyl chloride monomer is polymerized into poly(vinyl) chloride ("PVC"), a widely used polymer.

Catalysts and processes for the production of chlorinated hydrocarbons by oxychlorination have been well established for a number of years. Specifically, oxychlorination of ethylene with oxygen and hydrochloric acid in the presence of a catalyst to produce 1,2-dichloroethane, is widely practiced in commercial installations throughout the world.

The catalyst composition, that is, the catalyst itself and its support material, can be in the form of a fluidized bed of particles which are fluidized during the reaction, or in the form of a fixed bed of particles.

For example, a fluidized bed process for oxychlorination of ethylene to produce ethylene dichloride, involves the vapor phase reaction, over a fluidized catalyst bed, of a mixture of ethylene, hydrogen chloride and oxygen or an oxygen containing gas.

The common fixed bed alternative method involves use of a fixed bed catalyst through which a mixture of ethylene, hydrogen chloride and oxygen or an oxygen containing gas is reacted to produce ethylene dichloride.

In the oxychlorination of ethylene with such a catalytic fixed bed, the catalysts advantageously contain copper II chloride on a carrier in combination with promoters, such as potassium chloride.

In the conduct of a fixed bed oxychlorination process, major concerns include control of the reaction temperature, catalyst selectivity and activity, and pressure drop.

Catalyst selectivity is of great economic importance, as it is desirable for the oxychlorination catalyst to effect the highest possible yield of ethylene dichloride based on ethylene (that is, for the ethylene to be more completely converted to ethylene dichloride, with less ethylene being reacted to carbon oxides or higher chlorinated materials). In the high volume business of manufacturing ethylene dichloride, small increases in the efficiency of ethylene conversion to ethylene dichloride are very valuable. Further, increased ethylene efficiency reduces the amount of by-products produced and the associated potential of release of hydrocarbons and chlorinated hydrocarbons to the environment.

It is particularly desirable, when carrying out the oxychlorination of ethylene with a catalytic fixed bed method that the temperature be controlled to avoid damage to the catalyst, that the pressure drop caused by the catalyst be low, that the effective surface of the catalyst be large, and that the heat conductivity between the catalyst and an optional inert diluting agent be good.

The oxychlorination reaction itself is highly exothermic and in addition, control of the reaction temperature is hampered by the fact that the catalyst bed itself has a low heat conductivity. As a result of these two factors, there is a danger of the formation of undesirably high localized temperature zones in the catalyst bed which damage the catalyst and reduce the selectivity.

Thus, numerous expedients have been proposed in the art aimed at preventing or at least minimizing the existence of such exceptionally high localized temperatures.

For example, it has been variously proposed to control temperature by adjusting the ratio of the reactants, by diluting the feed with an inert gas or an excess of one or more of the reactants, by utilizing a tubular reactor with controlled external cooling and/or tubes of varying diameters, by diluting the catalyst particles with inert particles, and by varying the particle size of the catalyst and/or inert particles.

As for the pressure drop, the use of formed carrier catalysts generally results in a very high pressure drop as the hollow spaces in a densely packed bed are very small. Unfortunately, lowering the pressure drop by means of enlarging the dimensions of diameter and length of the carrier bodies, usually results in small conversions since this reduces the active surface of the catalyst.

U.S. Patent No. 4,510,263, issued April 9, 1985 to Pereira et al., discloses a catalyst which is cylindrical with an annular configuration having internal vanes or ribs extending from the inner wall to the center of the extrudate particle, with an outside diameter of up to 6.5 millimeters (mm), with vane thickness of 0.10 to 0.30 of the diameter.

U.S. Patent No. 5,166,120, issued November 24, 1992 to Deller et al., discloses a hollow cylindrical gamma alumina oxychlorination catalyst having a 4 millimeter to 6 millimeter outer diameter and height 1.7 to 3.75 times the outer diameter. Further disclosed carrying out oxychlorination in a series of several reactors, with oxygen distributed over the reactors, and with the reactors being filled with catalysts of differing activity stages which increase in the direction of flow.

EP 0146925 filed December 19, 1984 discloses a method for the oxychlorination of ethylene. In two-reactor installations, the total quantity of ethylene and hydrogen chloride is fed to the first reactor while the oxygen is divided between the two reactors. This reference requires utilization of three or more reactors in order to divide both hydrogen chloride and oxygen between the first two serially connected reactors.

DE 1468465 filed July 26, 1962 discloses oxychlorination of hydrocarbons by passing gaseous hydrocarbon, oxygen and hydrogen chloride through a first reactor over a fluid-bed catalyst and then through a second reactor over a solid bed catalyst. Example 2 discloses oxychlorination of ethane to ethylene dichloride by feeding ethane, oxygen and hydrogen chloride to a first reactor heated to 500°C to form primarily ethylene which is then fed to a second reactor with O₂ and HCl to form primarily ethylene dichloride.

There is a need in the art for an improved catalyst for and method of oxychlorinating ethylene.

It is an object of the present invention to provide for an improved catalyst for and method of oxychlorinating ethylene.

This and other objects of the present invention will become apparent to those of skill in the art upon review of this patent specification, including its drawings and claims.

The catalyst includes a support having deposited thereon an active metal composition comprising from 1 weight percent to 12 weight percent copper, and from 0.2 weight percent to 5 weight percent alkali metal, all weight percents based on the total dry weight of the catalyst. The shape of the support comprises a cylindrical hollow configuration with internal reinforcing vanes. The dimensions of the catalyst includes a diameter in the range of greater than 6.5 millimeters, a length in the range of 0.5 to 2 times the diameter, a wall thickness in the range of 0.1 to 0.3 times the diameter, and a vane thickness of 0.1 to 0.3 times the diameter.

There is provided a method of oxychlorinating ethylene into ethylene dichloride. The method includes contacting ethylene, oxygen and hydrogen chloride together in the presence of a catalyst to produce ethylene dichloride, wherein the catalyst is the catalyst described above.

According to the present invention there is provided a method of oxychlorinating ethylene into ethylene dichloride. The method includes utilizing no more than two oxychlorination reactors and splitting the oxygen and hydrogen chloride feeds between the reactors. The method also includes feeding ethylene, hydrogen chloride and oxygen to the first reactor where the ethylene, hydrogen chloride and oxygen are contacted together in the presence of an copper chloride alumina-supported catalyst to produce a first reactor product stream. The method further includes feeding, hydrogen chloride, oxygen, and the first reactor product stream, to the second reactor where the ethylene, hydrogen chloride, oxygen and first reactor stream are contacted together in the presence of an alumina catalyst to produce a second reactor product stream. Optionally ethylene feed may also be split between the reactors. Additionally, recycle streams may be utilized and split between the reactors.

The mol ratio of the hydrogen chloride fed to the first reactor to the hydrogen chloride fed to the second reactor is in the range of 50:50 to 99:1, and wherein the mol ratio of the oxygen fed to the first reactor to the oxygen fed to the second reactor is in the range of 50:50 to 99:1. The second reactor product stream may then be subjected to further processing to recover the ethylene dichloride product as is well known in the oxychlorination art, including water quenching and caustic washing.

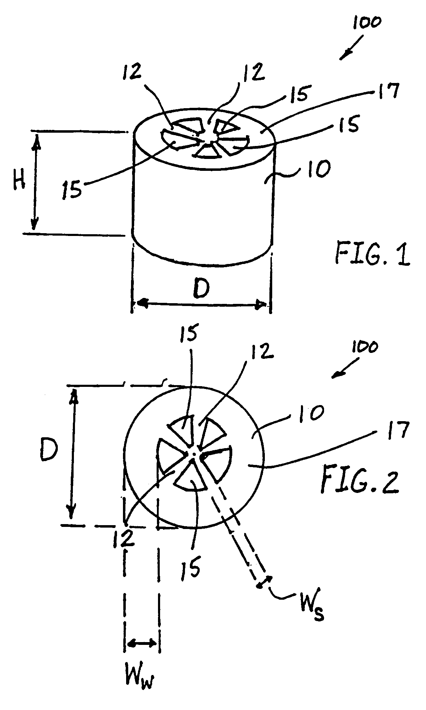

The oxychlorination catalyst of the present invention is an alumina support impregnated with a copper catalyst. The oxychlorination catalyst is fabricated in the form of a small tubular extruded member having a series of vanes or reinforcing members which extend through the center of the axis of rotation of the tubular member. Viewed from the top or bottom in cross-section, the vanes appear to extend from the center as a series of ribs which extend out to the tubular element. This spoked wagon wheel shape is better understood by reference to Figures 1 and 2.
Figure 1 is an illustration of one embodiment of catalyst 100 of the present invention showing partially hollow cylindrical catalyst support 10 of height H and diameter D, having wall 17, spokes 12 and passages 15.
Figure 2 is a view from the top or bottom of catalyst 100 of Figure 1, showing in cross-section, partially hollow cylindrical catalyst support 10 having wall 17 of width W_{w}, spokes 12 of width Wₛ, and passages 15.

The diameter D of the catalyst of the present invention is greater than 6.5 millimeters. Preferably, the diameter D of the catalyst of the present invention ranges from 6.5 millimeters to 10 millimeters. More preferably, the diameter D of the catalyst of the present invention ranges from 7 millimeters to 10 millimeters, even more preferably ranges from 7 millimeters to 9 millimeters and most preferably ranges from 8 millimeters to 9 millimeters.

The height H of the catalyst of the present invention may be any suitable height, depending upon the operating conditions, provided that the desired oxychlorination is achieved. Advantageously, the ratio of the height H to diameter D ranges from 0.5 to 5. Preferably, the ratio of the height H to diameter D ranges from 0.7 to 1.5, and more preferably from 0.8 to 1.2. Most preferably the ratio of the height H to diameter D is 1.

The wall width W_{w} of the catalyst of the present invention may be any suitable width, depending upon the operating conditions. Advantageously, the ratio of the wall width W_{w} to the diameter D ranges from 0.1 to 0.3, preferably from 0.2 to 0.25.

The spoke width Wₛ of the catalyst of the present invention may be any suitable width, depending upon the operating conditions. Advantageously, the ratio of the spoke width Wₛ to the diameter D ranges from 0.1 to 0.3, preferably from 0.15 to 0.25, and more preferably from 0.2 to 0.25.

In the embodiment shown in Figures 1 and 2, catalyst 100 is shown as having five spokes 12. However, it is to be understood that in the practice of the present invention, any suitable number of spokes 12 can be utilized provided that the desired oxychlorination is achieved. As the number of spokes 12 increases, the passages 15 become smaller in cross-sectional area, thereby increasing the pressure drop of operating the oxychlorination reaction. The number of spokes 12 will advantageously range from 2 to 12, preferably from 3 to 8, more preferably from 4 to 6, and is most preferably 5.

Catalyst support 10 of the present invention will conveniently comprise alumina, with virtually no impurities. Alumina support materials for oxychlorination catalysts are widely commercially available. The catalyst support 10 may be an α alumina, γ alumina, the so-called microgel aluminas or other forms of "activated" alumina. Preferably the support is a γ alumina.

The aluminas used in preparing catalyst support 10 are preferably first activated by heating at a suitable elevated temperature at which the alumina is dehydrated, as is otherwise known in the art. For instance, such activation can be conducted at 400 degrees Celsius to 600 degrees Celsius (°C). However, unactivated aluminas can also be utilized.

Methods of fabricating an alumina support into a desired geometric shape is well known to those of skill in the art. The method of fabricating catalyst support 10 of the present invention into the desired geometric shape is not critical, and any suitable method may be employed, including extrusion, pressing and molding. Preferably, catalyst support 10 is fabricated by extrusion due to increased crush strength properties, and the lower cost of manufacturing utilizing extrusion.

Methods for preparing a copper oxychlorination catalyst from alumina supports and CuCl₂ are well known to those of skill in the art. In the practice of the present invention, the method of preparation of the catalyst composition is not in itself critical, and any suitable method may be utilized. A simple technique is the incipient wetness method which comprises impregnating the support in a single stage with an aqueous solution containing the required quantities of CuCl₂ and any other desired metal. The aqueous solution is applied to the carrier and becomes absorbed. The impregnated support is then filtered if necessary and finally dried. Filtration will not be necessary if the support is contacted with a volume of the aqueous solution which is not more than sufficient to saturate the support.

Many metals have been utilized with copper as an oxychlorination catalyst, and any other suitable metal may be utilized in the catalyst of the present invention. Non-limiting of other metals which may optionally be utilized in the catalyst of the present invention include alkali metals, alkaline earth metals, rare earth metals, and Group II metals. Such metals include alkali metal chlorides and lanthanide oxides. These additional metals are applied either as metal salts, oxides or chlorides as is well known in the art. A preferred alkali metal chloride is potassium chloride which is very commonly recognized as a promoter, and is preferably utilized in the catalyst of the present invention.

It is to be understood that the metal oxides absorbed into the catalyst can exist either in an anhydrous state in the catalyst composition or, if desired, in the form of their hydrates. The later situation will occur, for example, if the drying conditions employed when using an aqueous support impregnation technique are insufficient to drive off the water of crystallization from the chlorides.

The oxychlorination catalyst of the present invention will generally comprise a suitable amount of copper to provide the desired catalytic effect. The copper is typically burdened as cupric chloride. Copper will commonly comprise in the range of 1 weight percent to 12 weight percent of the dry weight of the catalyst, preferably in the range of 1 weight percent to 8 weight percent of the dry weight of the catalyst. When utilized, an alkali metal, preferably potassium, will commonly comprise in the range of 0.2 weight percent to 5 weight percent of the dry weight of the catalyst, preferably in the range of 0.8 weight percent to 3 weight percent of the dry weight of the catalyst.

The average BET pore diameter of the catalyst of the present invention is advantageously greater than 80 Angstroms (Å). One Angstrom equals 10⁻¹⁰ meter. Conveniently, the average pore diameter of the catalyst utilized in the present invention will be in the range of 80 Å to 150 Å. Preferably, the average pore diameter is in the range of 90 Å to 140 Å, and most preferably in the range of 110 Å to 130 Å.

The BET pore volume of the catalyst of the present invention is advantageously greater than 0.7 cubic centimeters per gram (cc/g). An advantageous range of the pore volume of the catalyst of the present invention is from 0.3 cc/g to 0.9 cc/g. Preferably, the catalyst will have a pore volume in the range of 0.4 to 0.8 cc/g, more preferably in the range of 0.5 to 0.7 cc/g.

In the practice of the present invention, a high BET surface area catalyst support is utilized. The surface area of the catalyst will advantageously be in the range of 50 meters squared per gram (m²/g) to 380 m²/g. Preferably, the surface area of the catalyst of the present invention is in the range of 100 m²/g to 160 m²/g, and most preferably in the range of 120 m²/g to 140 m²/g.

The catalyst of the present invention is generally utilized in a fixed bed oxychlorination system, and can be employed in any suitable oxychlorination reaction scheme, using general techniques and reaction conditions well known to those of skill in the art. A preferred method of the present invention into which the catalyst of the present invention may be advantageously incorporated, utilizes a split feed of the oxygen and hydrogen chloride reactors to a two reactor oxychlorination scheme.

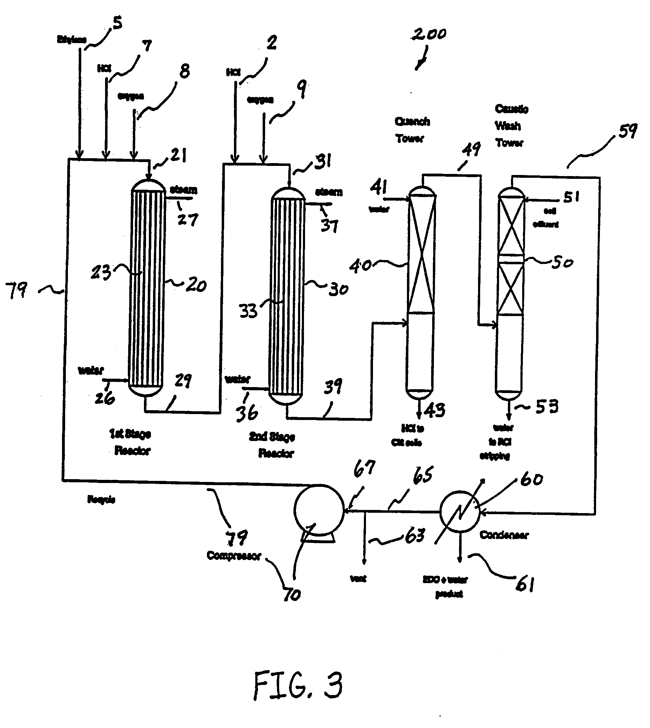

The method of the present invention is better understood by reference to Figure 3, which is a schematic representation of one embodiment of the oxychlorination process of the present invention, showing first reactor 20, second reactor 30, quench tower 40, caustic wash tower 50, condenser 60 and recycle pump 70.

Tube type reactors, with catalyst in the tubes and a cooling medium provided to the outside of the tubes are well known as being suitable for oxychlorination. While first reactor 20 and second reactor 30 may be any suitable types of reactors, they are preferably tube reactors having catalyst within a multiplicity of tubes 23 and 33, respectively. Advantageously, tubes 23 and 33 have diameters in the range of 0.5 inch to 3 inches (13 to 80 millimeters), with diameters in the range of 1.25 to 2.0 inches (32 to 50 millimeters) not uncommon. Preferably, the diameters will be in the range of 1.5 inches to 2.0 inches (38 to 50 millimeters). Tube lengths effectively range from 20 to 50 feet (6 to 15 meters), preferably from 20 to 40 feet (6 to 12 meters), and are most preferably 30 feet (9 meters).

The exothermic heat of reaction is removed utilizing a heat transfer media applied to the shell side of first reactor 20 and second reactor 30. As shown for reactor 20, the medium enters in stream 26 and exits through stream 27. Similarly for reactor 30, the medium enters in stream 36 and exits through stream 37. As the type of heat transfer media is not critical, any suitable media known to those of skill in the art may be utilized. Examples of suitable heat transfer are high boiling mineral oils, silicon oils, and water. It is preferred to utilize water, which is converted into medium-pressure steam by adsorption of heat. As shown in Figure 3, water streams 26 and 27 are provided to reactors 20 and 30, respectively, and exit reactors 20 and 30 as steam streams 27 and 37, respectively.

In the practice of the method of the present invention, the hydrogen chloride and oxygen feeds are split between first reactor 20 and second reactor 30.

As shown in Figure 3, feed streams of ethylene 5, HCl 7 and oxygen 8, as well as recycle stream 79 are fed to first reactor 20 as feed stream 21. The resultant product stream 29 of first reactor 20 is combined with hydrogen chloride stream 2 and oxygen stream 9 and fed to second reactor 30 as feed stream 31. The molecular oxygen introduced into the reactors 20 and 30 may be introduced as such or in the form of an oxygen-containing gas mixture such as air.

The total amounts of ethylene, hydrogen chloride, and oxygen provided to both reactors, will advantageously be such that based on the amount of hydrogen chloride, a slight stoichiometric excess of ethylene and oxygen are provided. Such excess is conveniently in the range of 1 to 5 mole %, preferably 2 to 4%.

The hydrogen chloride utilized is split between feed stream 7 to first reactor 20 and feed stream 2 to the second reactor 30. In the practice of the present invention, the total hydrogen chloride provided is split between the first reactor 20 and the second reactor 30 in a volume ratio in the range of 50:50 to 99:1. Preferably, the total hydrogen chloride provided is split between the first reactor 20 and the second reactor 30 in a volume ratio in the range of 50:50 to 80:20, and most preferably in the range of 60:40 to 70:30.

The oxygen utilized is split between feed stream 8 to first reactor 20 and feed stream 9 to the second reactor 30. In the practice of the present invention, the total oxygen provided is split between the first reactor 20 and the second reactor 30 in a volume ratio in the range of 50:50 to 99:1. Preferably, the total oxygen provided is split between the first reactor 20 and the second reactor 30 in a volume ratio in the range of 50:50 to 80:20, and most preferably in the range of 60:40 to 70:30.

Contacting times within the reactors 20 and 30 will be any suitable to provide the desired oxychlorination. "Contacting time" is defined as the ratio of the free or usable volume of the reactor to the volumetric flow rate of the feed gases at the operating conditions. Contacting times are generally dictated by economics and the desired conversion, but are conveniently no more than a few minutes. Contacting times will preferably range from 2 to 6 seconds, and are more preferably in the range of 3 to 5 seconds.

The operating temperatures within first and second reactors 20 and 30 will advantageously range from 180°C to 350°C, preferably in the range of 200°C to 270°C.

The catalyst in reaction tubes 23 and 33, have in the direction of flow, gradually rising copper concentration within sections of the tubes, thus ensuring better distribution of heat over the whole catalyst and better dissipation of reaction heat as a consequence.

For example when using copper and potassium as the catalysts in reactors 20 and 30, the ratio of potassium to copper in the first third of the reactor is advantageously in the range of 1.2 to 0.8, in the second third in the range of 0.3 to 0.8, and in the last third in the range of 0.05 to 0.3.

Once the reactants proceed through first reactor 20 and second rector 30, the resultant product stream 39 may be processed in any suitable manner as is known in the art for recovery of the ethylene dichloride. Such known processing advantageously includes water quenching and caustic washing.

Referring again to the embodiment of Figure 3, product stream 39 from second reactor 30 is fed to quench tower 40, wherein product stream 39 is cooled by contact with water stream 41. Unconsumed hydrogen chloride and the bulk of the reaction water are condensed and exit though the bottom of quench tower 40 as bottoms stream 43. Uncondensed gases exit quench tower 40 through stream 49 to be contacted with caustic stream 51 in caustic wash tower 50. The water stream 53 is further processed by RCl stripping to remove the chlorinated organics.

Uncondensed gas stream 59 is condensed at condenser 60 to remove a water and ethylene dichloride product stream 61. Uncondensed stream 65, containing ethylene; carbon monoxide and dioxide, and small amounts of ethylene dichloride as well as other contaminants, is first vented to remove gases as vent stream 63, and then stream 67 is recycled back to first reactor 20 as recycle stream 79 using compressor 70.

While the above process has been illustrated with reference to the catalyst of the present invention, it is to be understood that the particular catalyst composition and shape is not critical, and that any suitable catalyst, having a range of catalytic compositions, and having a range of effective shapes may be utilized. Accordingly, in the practice of the present invention, any oxychlorination catalyst composition may be utilized. Additionally, a variety of advantageous catalyst shapes may be utilized, including as nonlimiting examples, spheres, columns, rings, and annular columns. Preferable alternate geometric shapes suitable for use in the method of the present invention includes rings and annular columns having the same height, diameter and wall thickness ranges as the catalyst of the present invention.

### Example 1 Comparison of Penta-Rings and Typical Spherical Catalyst

The experiments were carried out in a Berty CSTR reactor loaded with oxychlorination catalyst loaded with equal amounts of Copper and Potassium on both supports. Gaseous reactants were fed to the reactor at pre-set ratios, as shown in Table 1. Catalyst content was varied between Penta-Rings and spheres to match HCl conversion at 240°C. The temperature was then increased to 290°C. The reactants were fed to the reactors through thermal mass flow meters into the reaction chamber where the catalyst is held in a small basket with screen on top and bottom of the catalyst. Within the reactor and directly underneath the catalyst basket lies a rotating impeller to continuously mix the gas. These "Berty" reactors are commonly used in catalysts to measure catalyst kinetics.

**Table 1**

| Experimental Conditions | |
|---|---|
| Temperature | 240°C, 290°C |
| HCl feed, mol/hr | 1.0 |
| C₂H₄ feed, mol/hr | 0.5 |
| Oxygen feed, mol/hr | 0.2 |
| Impeller rotations per minute (RPM), | 2000 |
| Pressure, psig | 40 (276 kPa) |
| Catalyst weight, g | 8.47 for Penta-Ring #1, 10.0 for sphere, and 9.11 for Penta-Ring #2 |
| Catalyst surface area, (m²/g) | 220 for Penta-Ring #1, 230 for sphere and 120 for Penta-Ring #2 |

The product was condensed and the organic and aqueous phases separated. Analysis of the vent gases and organic phase were carried out with a gas chromatograph. Unreacted HCl was absorbed in a wash bottle of water and then titrated, as was the condensed aqueous phase. A comparison of EDC selectivities and major by-products with Penta-Rings and spheres is given in Table 2.

**Table 2**

| Comparison of Penta-Rings and Spheres | | | | | | |
|---|---|---|---|---|---|---|
| | Sphere, 240°C | Penta-Ring #1, 240°C | Penta-Ring #2, 240°C | Sphere 290°C | Penta-Ring #1, 290°C | Penta-Ring #2, 290°C |
| HCl Conversion, % | 30.6 | 31.7 | 31.4 | 47.6 | 48.4 | 52.5 |
| EDC Selectivity, % | 98.6 | 99.2 | 99.1 | 96.0 | 96.8 | 97.9 |
| Ethyl Chloride, % | 0.51 | 0.32 | 0.55 | 0.38 | 0.34 | 0.40 |
| 1,1,2-Trichloroethane, % | 0.25 | 0.12 | 0.08 | 1.08 | 0.63 | 0.59 |
| CO, % | 0.18 | 0.11 | 0.07 | 1.03 | 1.09 | 0.44 |
| CO2, % | 0.14 | 0.05 | 0.04 | 1.09 | 0.63 | 0.29 |
| Balance | 0.32 | 0.20 | 0.16 | 0.42 | 0.51 | 0.38 |

### Example 2

Operation of the process described can include but is not limited to the catalyst described in this invention. An example of the process operating with a spherical catalyst with composition similar to the one described in the invention is given as an illustration. For the catalysts referred to in this example as catalysts A, B, and C, the weight percents of copper chloride and potassium chloride are 3.6/1.8%, 3.6/0.7%, and 6.0/0.2%, respectively.

The two reactors each consisted of tubes 1.5 inches (38 millimeters) in outside diameter. Heat generated in the reaction was removed through the generation of steam in a shell that encased the tubes. The reactors are each divided into 3 sections of 10 feet (3 meters) in length. Catalyst A was used in sections A and B in volumetric concentrations of 55 and 75% respectively. A mixture of Catalyst A and B in the volumetric ratio of 1.271/1 was used in the bottom section of the first reactor. This first section of the second reactor utilized Catalyst A and B as well, this time in a ratio of 0.55/1. The second section of the second reactor utilized Catalysts B and C in the ratio of 0.67/1 and Catalyst C was used in the last section of the second reactor.

Feeds of ethylene, oxygen and HCl were initiated to the reactor system and the system was allowed to stabilize before data was collected. The operating data at what are considered optimal conditions are listed below:

| | Run #1 | Run #2 |
|---|---|---|
| HCl flow (gmol/sec) | 0.45 | 0.45 |
| System Pressure (psig) | 50 (350 kPa) | 50 (350 kPa) |
| O₂ excess (%) | 7.6 | 7.6 |
| C₂H₄/HCl (mol/mol) | 0.42 | 0.42 |
| HCl split (R¹/R²) | 0.5/0.5 | 0.5/0.5 |
| O₂ split (R¹/R²) | 0.5/0.5 | 0.5/0.5 |
| % C₂H₄ oxidized | 1.281 | 2.163 |
| % C₂H₄ to EtCl | 0.422 | 0.490 |
| Hot Spot Temperature R¹ | 267°C | 294°C |
| Hot Spot Temperature R² | 232°C | 227°C |
| HCl conversion (%) | 95.6 | 91.8 |

In both cases above coolant temperatures were maintained between 205 and 212°C. Under these conditions the process was able to maintain low process temperatures and also excellent reactor performance.

While the illustrative embodiments of the invention have been described with particularity, it will be understood that various other modifications will be apparent to and can be readily made by those skilled in the art without departing from the spirit and scope of the invention. Accordingly, it is not intended that the scope of the claims appended hereto be limited to the examples and descriptions set forth herein but rather that the claims be construed as encompassing all the features of patentable novelty which reside in the present invention, including all features which would be treated as equivalents thereof by those skilled the art to which this invention pertains.

## Claims

1. A method of oxychlorination utilizing no more than two oxychlorination reactors, the method comprising:
(a) feeding ethylene, hydrogen chloride and oxygen to a first reactor where the ethylene, hydrogen chloride and oxygen are contacted together in the presence of a copper containing catalyst to produce a first reactor product stream; and
(b) feeding hydrogen chloride, oxygen and the first reactor product stream to a second reactor where the hydrogen chloride, oxygen and first reactor stream are contacted together in the presence of an alumina catalyst to produce a second reactor product stream,
wherein the mol ratio of the hydrogen chloride fed to the first reactor to the hydrogen chloride fed to the second reactor is in the range of 50:50 to 99:1, and wherein the mol ratio of the oxygen fed to the first reactor to the oxygen fed to the second reactor is in the range of 50:50 to 99:1.

2. The method of claim 1 further comprising:
(c) contacting the second reactor product stream with water to form a quenched product stream.

3. The method of claim 2 further comprising:
(d) contacting the quenched product stream with a caustic to form a washed stream;
(e) condensing the washed stream to form an ethylene dichloride product stream and a recycle gas stream; and
(f) recycling the recycle gas stream to the first reactor.

4. The method of claim 1, 2 or 3 wherein the mol ratio of the hydrogen chloride fed to the first reactor to the hydrogen chloride fed to the second reactor is in the range of 50:50 to 80:20, and wherein the mol ratio of the oxygen fed to the first reactor to the oxygen fed to the second reactor is in the range of 50:50 to 80:20.

5. The method of claim 1, 2 or 3 wherein the copper containing catalyst comprises an alumina support having deposited thereon an active metal composition comprising from 1 weight percent to 12 weight percent copper, and from 0.2 weight percent to 5 weight percent alkali metal, all weight percents based on the total dry weight of the catalyst, and wherein the support comprises a cylindrical hollow configuration with internal reinforcing vanes, and a diameter in the range of greater than 6.5 mm.

6. The method of claim 5 wherein the support has a length in the range of 0.5 to 2 times the diameter.

7. The method of claim 5 wherein the diameter is in the range of 7 mm to 9 mm.

8. The method of claim 5 wherein the support has a surface area in the range of 50 m²/g to 380 m²/g.

## Patentansprüche

1. Verfahren zur Oxychlorinierung unter Verwendung von nicht mehr als zwei Oxychlorinierungsreaktoren, wobei das Verfahren umfaßt:
(a) Zuführen von Ethylen, Chlorwasserstoff und Sauerstoff in einen ersten Reaktor, worin Ethylen, Chlorwasserstoff und Sauerstoff in Gegenwart eines kupferhaltigen Katalysators miteinander in Kontakt gebracht werden, um einen Produktstrom des ersten Reaktors zu erzeugen, und
(b) Zuführen von Chlorwasserstoff, Sauerstoff und des Produktstroms des ersten Reaktors in einen zweiten Reaktor, worin Chlorwasserstoff, Sauerstoff und der Strom aus dem ersten Reaktor in Gegenwart eines Aluminiumoxidkatalysators miteinander in Kontakt gebracht werden, um einen Produktstrom des zweiten Reaktors zu erzeugen,
wobei das Molverhältnis von Chlorwasserstoff, der dem ersten Reaktor zugeführt wird, zu dem Chlorwasserstoff, der dem zweiten Reaktor zugeführt wird, im Bereich von 50:50 bis 99:1 liegt und wobei das Molverhältnis von Sauerstoff, der dem ersten Reaktor zugeführt wird, zu dem Sauerstoff, der dem zweiten Reaktor zugeführt wird, im Bereich von 50:50 bis 99:1 liegt.

2. Verfahren nach Anspruch 1, weiterhin umfassend:
(c) Inkontaktbringen des Produktstroms des zweiten Reaktors mit Wasser, um einen gequenchten Produktstrom zu bilden.

3. Verfahren nach Anspruch 2, weiterhin umfassend:
(d) Inkontaktbringen des gequenchten Produktstrom mit einer Lauge, um einen gewaschenen Strom auszubilden,
(e) Kondensieren des gewaschenen Stroms, um einen Ethylendichloridproduktstrom und einen Recyclinggasstrom zu bilden, und
(f) Zurückführen des Recyclinggasstroms in den ersten Reaktor.

4. Verfahren nach Anspruch 1, 2 oder 3, wobei das Molverhältnis von Chlorwasserstoff, der dem ersten Reaktor zugeführt wird, zu dem Chlorwasserstoff, der dem zweiten Reaktor zugeführt wird, im Bereich von 50:50 bis 80:20 liegt und wobei das Molverhältnis von Sauerstoff, der dem ersten Reaktor zugeführt wird, zu dem Sauerstoff, der dem zweiten Reaktor zugeführt wird, im Bereich von 50:50 bis 80:20 liegt.

5. Verfahren nach Anspruch 1, 2 oder 3, wobei der kupferhaltige Katalysator einen Aluminiumoxidträger enthält, auf dem eine Zusammensetzung mit aktivem Metall, die 1 bis 12 Gew.-% Kupfer und 0,2 bis 5 Gew.-% Alkalimetall enthält, abgeschieden ist, wobei alle Gewichtsprozentangaben sich auf das Gesamttrockengewicht des Katalysators beziehen und wobei der Träger eine zylindrische hohle Konfiguration mit internen verstärkenden Wänden und einen Durchmesser im Bereich von größer als 6,5 mm aufweist.

6. Verfahren nach Anspruch 5, wobei der Träger eine Länge im Bereich des 0,5 bis 2fachen des Durchmessers aufweist.

7. Verfahren nach Anspruch 5, wobei der Durchmesser im Bereich von 7 mm bis 9 mm liegt.

8. Verfahren nach Anspruch 5, wobei der Träger eine Oberfläche im Bereich von 50 m²/g bis 380 m²/g aufweist.

## Revendications

1. Procédé d'oxychloration n'utilisant pas plus de deux réacteurs d'oxychloration, le procédé consistant:
(a) à introduire de l'éthylène, du chlorure d'hydrogène et de l'oxygène dans un premier réacteur où l'éthylène, le chlorure d'hydrogène et l'oxygène sont mis en contact en présence d'un catalyseur contenant du cuivre en vue de produire un courant de produit du premier réacteur; et
(b) à introduire du chlorure d'hydrogène, de l'oxygène et le courant de produit du premier réacteur dans un deuxième réacteur où le chlorure d'hydrogène, l'oxygène et le courant du premier réacteur sont mis en contact en présence d'un catalyseur sur alumine en vue de produire un courant de produit du deuxième réacteur;
dans lequel le rapport molaire de chlorure d'hydrogène alimentant le premier réacteur au chlorure d'hydrogène alimentant le deuxième réacteur est de 50:50 à 99:1, et dans lequel le rapport molaire de l'oxygène alimentant le premier réacteur à l'oxygène alimentant le deuxième réacteur est de 50:50 à 99:1.

2. Procédé selon la revendication 1, consistant de plus:
(c) à mettre le courant de produit du deuxième réacteur en contact avec de l'eau afin de former un courant de produit trempé.

3. Procédé selon la revendication 2, consistant de plus:
(d) à mettre le courant de produit trempé en contact avec une base caustique pour former un courant lavé;
(e) à condenser le courant lavé en vue de former un courant de produit de dichlorure d'éthylène et un courant de gaz de recyclage; et
(f) à recycler le courant de gaz de recyclage vers le premier réacteur.

4. Procédé selon l'une des revendications 1, 2 ou 3, dans lequel le rapport molaire du chlorure d'hydrogène alimentant le premier réacteur au chlorure d'hydrogène alimentant le deuxième réacteur est de 50:50 à 80:20, et dans lequel le rapport molaire de l'oxygène alimentant le premier réacteur à l'oxygène alimentant le deuxième réacteur est de 50:50 a 80:20.

5. Procédé selon l'une des revendications 1, 2 ou 3, dans lequel le catalyseur contenant du cuivre comporte un support d'alumine sur lequel est déposée une composition de métal actif comprenant 1% en poids à 12% en poids de cuivre, et 0,2% en poids a 5% en poids de métal alcalin, tous les pourcentages pondéraux étant rapportés au poids total sec du catalyseur, et dans lequel le support a une configuration creuse cylindrique avec des ailettes de renforcement internes, et un diamètre supérieur à 6,5 mm.

6. Procédé selon la revendication 5, dans lequel le support a une longueur de 0,5 a 2 fois son diamètre.

7. Procédé selon la revendication 5, dans lequel le diamètre est de 7 mm a 9 mm.

8. Procédé selon la revendication 5, dans lequel le support a une surface spécifique de 50 m²/g à 380 m²/g.
